# EUROPÄISCHE PATENTANMELDUNG

(11) **EP 0 700 891 A1**
(43) Veröffentlichungstag der Anmeldung: **13.03.1996**
(21) Anmeldenummer: 95112006.2
(22) Anmeldetag: 31.07.1995
(51) Int. Cl.: C07C 45/58

(54) **Verfahren zur Herstellung von alpha-Hydroxyketonen**

(30) Priorität: 08.08.1994 DE 4428047
(71) Anmelder: BASF AKTIENGESELLSCHAFT, D-67056 Ludwigshafen (DE)
(72) Erfinder: Rieber, Norbert, Dr., D-68259 Mannheim (DE); Teles, Joaquim Henrique, Dr., D-67059 Ludwigshafen (DE)

(57) **Zusammenfassung**

Verfahren zur Herstellung von α-Hydroxyketonen der allgemeinen Formel I 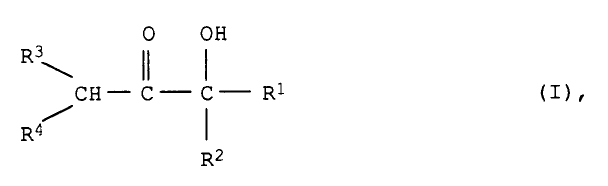
in der
- R¹,R²,R³,R⁴: Wasserstoff und C₁- bis C₈-Alkyl oder
- R¹ und R³: gemeinsam eine gegebenenfalls ein- bis dreifach durch Methyl und/oder Ethyl substituierte C₂- bis C₁₀-Alkylenkette und
- R⁵: Wasserstoff, Methyl und Ethyl
bedeuten, aus 2,3-Epoxialkoholen der allgemeinen Formel II in der R¹, R², R³, R⁴, R⁵ und n die obengenannten Bedeutungen haben, bei Temperaturen von (-10) bis 120°C und Drücken von 0,01 bis 20 bar in Gegenwart eines Katalysators, indem man als Katalysator eine Hydridokobaltverbindung einsetzt sowie die Herstellung von 2,3-Epoxialkohole II aus von 3-Hydroperoxialkenen III und deren Herstellung aus Alkenen IV mit Sauerstoff oder Sauerstoff enthaltenden Gasgemischen.

## Beschreibung

Die vorliegende Erfindung betrifft ein Verfahren zur Herstellung von α-Hydroxyketonen aus 2,3-Epoxialkoholen in Gegenwart eines Katalysators, wobei man die 2,3-Epoxialkohole durch Umsetzung von Alkenen mit Sauerstoff oder Sauerstoff enthaltenden Gasgemischen und Umlagerung des entstandenen Alkylhydroperoxids in Gegenwart eines Katalysators erhalten kann.

Die Umlagerung von Epoxiden in Ketone mit Magnesiumbromidetherat oder mit Bortrifluoridetherat als Katalysator ist aus J. Am. Chem. Soc. 77, 5083-5089 (1955) bekannt. Diese Katalysatoren sind entweder unwirksam oder katalysieren die Polymerisation des Epoxyalkohols.

Aus Bull. Chem. Soc. Japan, 48, 1337 bis 1338 (1975) ist die Umlagerung von Cyclohexenylhydroperoxid zu 2,3-Epoxycyclohexanol bekannt.

Die Oxidation von Cyclopenten zu Cyclopentenylhydroperoxid ist mit photochemisch angeregtem Sauerstoff in schlechten Ausbeuten aus Chem. Ber., 72, 1799 bis 1804 (1939) bekannt. Die unkatalysierte allylische Oxidation von Olefinen zu Allylhydroperoxiden ist aus Houben-Weyl, Bd. E13/1, Seite 64 bis 71 (1988) bekannt.

Der vorliegenden Erfindung lag die Aufgabe zugrunde, den zuvor genannten Nachteilen abzuhelfen.

Demgemäß wurde ein neues und verbessertes Verfahren zur Herstellung von α-Hydroxyketonen der allgemeinen Formel I 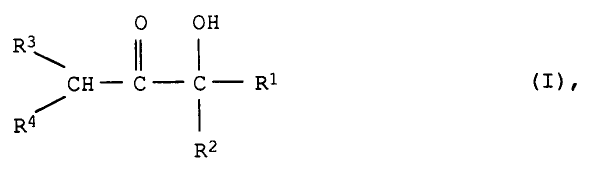
in der
- R¹,R²,R³,R⁴: Wasserstoff und C₁- bis C₈-Alkyl oder
- R¹ und R³: gemeinsam eine gegebenenfalls ein- bis dreifach durch Methyl und/oder Ethyl substituierte C₂- bis C₁₀-Alkylenkette und
- R⁵: Wasserstoff, Methyl und Ethyl
bedeuten, aus 2,3-Epoxialkoholen der allgemeinen Formel II 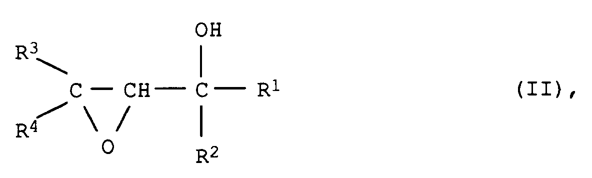
in der R¹, R², R³, R⁴, R⁵ und n die obengenannten Bedeutungen haben, bei Temperaturen von (-10) bisund 120°C und Drücken von 0,01 bis 20 bar in Gegenwart eines Katalysators gefunden, welches dadurch gekennzeichnet ist, daß man als Katalysator eine Hydridokobaltverbindung einsetzt sowie die Herstellung von 2,3-Epoxialkohole II aus von 3-Hydroperoxialkenen III und deren Herstellung aus Alkenen IV mit Sauerstoff oder Sauerstoff enthaltenden Gasgemischen.

Das erfindungsgemäße Verfahren läßt sich wie folgt durchführen:

### Isomerisierung von 2,3-Epoxycyclopentanol zu 2-Hydroxycyclopentanon

2,3-Epoxycyclopentanol II kann in Substanz oder gelöst in einem inerten Lösungsmittel durch Behandlung mit einer Hydridokobaltverbindung als Katalysator bei Temperaturen von (-10) bis 120°C, bevorzugt 20 bis 100°C, besonders bevorzugt 40 bis 80°C und Drücken von 0,01 bis 20 bar, bevorzugt 0,1 bis 5 bar, besonders bevorzugt Normaldruck (Atmosphärendruck) zu 2-Hydroxycyclopentanon isomerisiert werden.

Als Hydridokobaltverbindungen eignen sich solche der allgemeinen Formel HCoL₄, in der L für Liganden mit schwachen σ-Donor- und guten π-Akzeptoreigenschaften,bevorzugt für CO, Tri-C₁- bis C₈-alkylphosphite, Arylphosphite, PF₃ oder Tris-(pentafluorphenyl)-phosphin steht. Die Hydridokobaltverbindungen können auch in situ durch Reaktion vom Dicobaltoctacarbonyl mit protischen Verbindungen (z.B. Alkoholen oder Säuren, die als Lösungsmittel oder als Cokatalysator verwendet werden, Wasserspuren im aprotischen Lösungsmittel oder den Epoxyalkohol selbst) oder durch die Reaktion von Tetracarbonylcobaltate (z.B. Natriumtetracarbonylcobaltat) mit einer Säure (z.B. p-Toluolsulfonsäure) erzeugt werden. Bevorzugt wird aus Dicobaltoctacarbonyl das Hydridocobalttetracarbonyl in situ erzeugt.

Das Molverhältnis von Hydridokobaltverbindung zum 2,3-Epoxycyclopentanol II liegt in der Regel bei 0,001:1 bis 2:1, bevorzugt 0,005:1 bis 1:1, besonders bevorzugt 0,01:1 bis 0,5:1.

Als inerte Lösungsmittel eignen sich z.B. Alkohole wie C₁- bis C₈-Alkanole, bevorzugt C₁- bis C₄-Alkanole wie Methanol, Ethanol, n-Propanol und iso-Propanol, Benzol, Alkylaromaten wie Toluol, ortho-, meta-und para-Xylol, Carbonsäuren wie C₁- bis C₈-Carbonsäuren, bevorzugt C₁- bis C₄-Carbonsäuren wie Ameisensäure, Essigsäure und Propionsäure, Carbonsäureester wie C₂- bis C₁₂-Carbonsäureester, bevorzugt C₂- bis C₆-Carbonsäureester wie Ameisensäuremethylester, Ameisensäureethylester, Essigsäure-methylester, Essigsäureethylester, Propionsäuremethylester und Propionsäureethylester. Besonders bevorzugt werden Methanol und Benzol als Lösungsmittel.

### Isomerisierung von Cyclopentenylhydroperoxid zu 2,3-Epoxycyclopentanol

Cyclopentenylhydroperoxid III kann in Gegenwart einer löslichen Verbindung eines Übergangsmetalls der Gruppen IVB, VB und VIB bei Temperaturen von (-20) bis 120°C, bevorzugt 20 bis 100°C, besonders bevorzugt 40 bis 90°C und Drücken von 0,01 bis 20 bar, bevorzugt 0,1 bis 5 bar, besonders bevorzugt Normaldruck (Atmosphärendruck) zu 2,3-Epoxycyclopentanol isomerisiert werden.

Als Katalysatoren der Übergangsmetalle der Gruppen IVB, VB und VIB eignen sich Verbindungen der Elemente Titan, Zirkon, Hafnium, Vanadin, Niob, Tantal, Chrom, Molybdän und Wolfram, bevorzugt Titan-, Vanadium- und Molybdänverbindungen, besonders bevorzugt Vanadiumverbindungen. Gute Ergebnisse können zum Beispiel mit Vanadylacetylacetonat [VO(acac)₂] oder mit Vanadyltrialkoxylaten [VO(OR)₋₃], in der R für C₁- bis C₁₂-Alkyl, bevorzugt C₁- bis C₈-Alkyl, besonders bevorzugt C₁- bis C₄-Alkyl wie Methyl, Ethyl, n-Propyl, iso-Propyl, n-Butyl, iso-Butyl, sec.-Butyl und tert.-Butyl oder Aryl wie Phenyl, 2-Methoxyphenyl, 1-Naphthyl und 2-Naphthyl, bevorzugt Phenyl, insbesondere iso-Propyl und tert.-Butyl steht, erzielt werden.

Als inerte Lösungsmittel eignen sich z.B. chlorierte Kohlenwasserstoffe wie Dichlormethan, Chloroform, 1,1,1-Trichlormethan, Ether wie Tetrahydrofuran, Aromaten wie Benzol und Toluol oder Kohlenwasserstoffe wie Cyclohexan, Ester von C₁- bis C₄-Carbonsäuren mit C₁- bis C₄-Alkohole wie Ethylacetat oder C₂- bis C₁₂-Nitrile wie Acetonitril oder Benzonitril.

Das Molverhältnis von Katalysatoren der Übergangsmetalle der Gruppen IVB, VB und VIB zum Cyclopentenylhydroperoxid III liegt in der Regel bei 0,00001:1 bis 0,1:1, bevorzugt 0,0005:1 bis 0,05:1, besonders bevorzugt 0,001:1 bis 0,01:1.

Der Zusatz eines Wasserfängers wie z.B. wasserfreies Natriumsulfat kann die Reaktion beschleunigen. Der Zusatz eines Säurefängers wie z.B. Calcium- oder Bariumoxid kann die Bildung von Nebenprodukten reduzieren.

Die Reaktionszeit wird in der Regel so gewählt, daß ein Hydroperoxidumsatz zwischen 50 und 100 % erreicht wird. Um die Aufarbeitung zu erleichtern, wird die Reaktion vorzugsweise bis zu einem Umsatz >90 % gefahren.

### Oxidation von Cyclopenten zu Cyclopentenylhydroperoxid

Cyclopenten IV kann in Substanz oder als Gemisch mit weniger leicht oxidierbaren Substanzen mit Sauerstoff oder einem Sauerstoff enthaltenden Gasgemisch, wie Luft in einem geeigneten Reaktor, z.B. einer Blasensäule oder einem Rührkessel mit Begasungsrührer bei Temperaturen von 20 bis 120°C, bevorzugt 35 bis 90°C, besonders bevorzugt 40 bis 80°C zu Cyclopentenylhydroperoxid III oxidiert.

Der Druck ist für diese Reaktion kein kritischer Parameter. Der Gesamtdruck im Reaktor sollte in der Regel größer als der Dampfdruck der flüssigen Komponenten bei der Reaktionstemperatur sein.

Für diese Oxidation wird in der Regel kein Katalysator benötigt. Bei der Oxidation können Säurefänger, z.B. Alkali- bzw. Erdalkalicarbonate, Alkali- bzw. Erdalkalihydrogencarbonate oder Alkali- bzw. Erdalkalicarboxylate zugesetzt werden.

Die Reaktionsdauer bzw. Verweilzeit wird in der Regel so gewählt, daß der Cyclopentenumsatz zwischen 1 und 70 % liegt. Besonders bevorzugt werden Cyclopentenumsätze zwischen 10 und 20 %.

Für die weitere Verarbeitung wird zweckmäßigerweise das nicht umgesetzte Cyclopenten abgetrennt und das Cyclopentenyl-hydroperoxid entweder isoliert oder in einem inerten Lösungsmittel aufgenommen.

Die Substituenten R¹, R², R³, R⁴, R⁵ und der Index n in den Verbindungen I, II, III und IV haben folgende Bedeutungen:
R¹,R²,R³,R⁴ unabhängig voneinander
- Wasserstoff,
- C₁- bis C₈-Alkyl, bevorzugt C₁- bis C₄-Alkyl, besonders bevorzugt Methyl und Ethyl,
R⁵
- Methyl,
- Ethyl,
- besonders bevorzugt Wasserstoff,
R¹ und R³
- gemeinsam eine gegebenenfalls ein- bis dreifach durch Methyl und/oder Ethyl substituierte C₂- bis C₁₀-Alkylenkette.

### Beispiele

### Beispiel 1

### Diskontinuierliche Oxidation von Cyclopenten zu Cyclopentenylhydroperoxid mit Sauerstoff bei erhöhtem Druck

In einem 400 ml Glasautoklav mit Rührer, Sauerstoffzufuhr und Druckregelung wurden 150 g stabilisatorfreies Cyclopenten und 3 g Cyclopentenylhydroperoxid (ca. 80 %ig) eingefüllt. Der Autoklav wurde dann mit einem Ölbad auf die gewünschte Temperatur gebracht. Sauerstoff wurde dann druckgeregelt zudosiert. Nach der vorgegebenen Zeit wurden die Versuche abgebrochen und der Reaktorinhalt analysiert. Der Einfluß von Temperatur und Druck auf Umsatz und Selektivität sind in den folgenden Tabellen angegeben.

**Tabelle 1**

| Einfluß von Reaktionstemperatur und Reaktionszeit bei konstantem Druck (P = 6 bar) | | | | | | |
|---|---|---|---|---|---|---|
| | T = 60°C | | T = 70°C | | T = 80°C | |
| Zeit/h | Umsatz/% | Selekt./% | Umsatz/% | Selekt./% | Umsatz/% | Selekt./% |
| 1 | | | 4,7 | 79,6 | 8,8 | 83 |
| 2 | | | 8,8 | 74,4 | 27,2 | 73,4 |
| 3 | | | 15,4 | 82,9 | 42,5 | 65,2 |
| 4 | 3,6 | 91,1 | 23,2 | 80,4 | 51,3 | 56,2 |

**Tabelle 2**

| Einfluß des Gesamtdrucks bei konstanter Temperatur (70°C) und Reaktionszeit (4H) | | |
|---|---|---|
| Druck/bar | Umsatz/% | Selekt./% |
| 4 | 23,0 | 84,0 |
| 6 | 23,2 | 80,4 |
| 8 | 21,4 | 81,8 |

### Beispiel 2

### Kontinuierliche Oxidation von Cyclopenten zu Cyclopentenyl-hydroperoxid mit Sauerstoff bei Normaldruck

Eine 1l-Blasensäule mit Doppelmantel und aufgesetztem Rückflußkühler wurde mit ca. 750 ml voroxidiertem Cyclopenten (ca. 15 Gew.-% Cyclopentenylhydroperoxid) gefüllt, auf ca. 40°C temperiert und mit einem konstanten Sauerstoffstrom (5 l/min) oxidiert. Durch eine Öffnung im unteren Viertel der Blasensäule wurde frisches, stabilisatorfreies Cyclopenten mit einer HPLC-Pumpe kontinuierlich zudosiert. Das Produkt wurde durch einen Überlauf im oberen Teil der Blasensäule kontinuierlich entnommen. Bei einem Cyclopentendurchsatz von 31,5 ml/h enthält das Produkt 19 bis 20 Gew.-% Cyclopentenylhydroperoxid (entspricht U∼ 16 % und S∼ 88 %). Das unumgesetzte Cyclopenten kann bei Raumtemperatur mit einem Rotationsverdampfer abgezogen werden. Der Rückstand enthält ca. 90 Gew.-% Hydroperoxid als farblose Flüssigkeit. Die restlichen 10 % bestehen zum großen Teil aus Cyclopentenon und Cyclopentenol. Das abdestillierte Cyclopenten ist nahezu rein und kann wieder in die Blasensäule eingespeist werden.

### Beispiel 3

### Oxidation von Cyclohexen zu Cyclohexenylhydroperoxid mit Sauerstoff bei Normaldruck in einer Blasensäule

Eine 5l-Blasensäule mit Doppelmantel und aufgesetztem Rückflußkühler wurde mit ca. 3 l Cyclohexen gefüllt, auf ca. 80°C temperiert und mit einem konstanten Sauerstoffstrom (10 l/min) oxidiert. Nach 7 Stunden war der Hydroperoxidgehalt auf 9,2 % angestiegen und die Säurezahl betrug 0,1 g KOH/g. Nach weiteren 3,5 Stunden war der Hydroperoxidgehalt auf 17,1 % angestiegen und die Säurezahl auf 0,74 g KOH/g. Der Austrag wurde mit 10 %iger Natriumcarbonatlösung gewaschen (Säurezahl nach der Wäsche: 0,15 g KOH/g), die organische Phase über Natriumsulfat getrocknet und eingeengt. Als Rückstand verbleiben 513 g rohes Cyclohexenylhydroperoxid (Gehalt: ca. 75 %, titrimetrisch). Dieses Hydroperoxid kann direkt für den zweiten Schritt verwendet werden.

### Beispiel 4

### Katalysatoren für die Umlagerung von Cyclopentenylhydroperoxid zu 2,3-Epoxycyclopentanol

In einem 1l-Dreihalskolben wurde den Katalysator in 100 g Lösungsmittel (siehe Tabelle 3) gelöst und zum refluxieren gebracht. Durch einen Tropftrichter wurde dann innerhalb einer Stunde eine Lösung von 50 g Cyclopentenylhydroperoxid (als rohes Hydroperoxid mit einem Gehalt von ca. 85 bis 95 %) in 50 g Lösungsmittel zugetropft. Die Umsetzung wurde durch Titration des Hydroperoxids verfolgt. In der Regel wurde die Reaktion abgebrochen, wenn der Hydroperoxidgehalt kleiner als 1 % war. Der Gehalt an Produkt im Reaktionsaustrag wurde mittels quantitativem GC bestimmt und der Austrag dann in einem Fallfilmverdampfer destilliert. Das Destillat (frei von Katalysator und Schwersieder) kann noch fein destilliert werden. Die Ergebnisse, die mit verschiedenen Katalysatoren erzielt wurden, sind in Tabelle 3 zusammengefaßt.

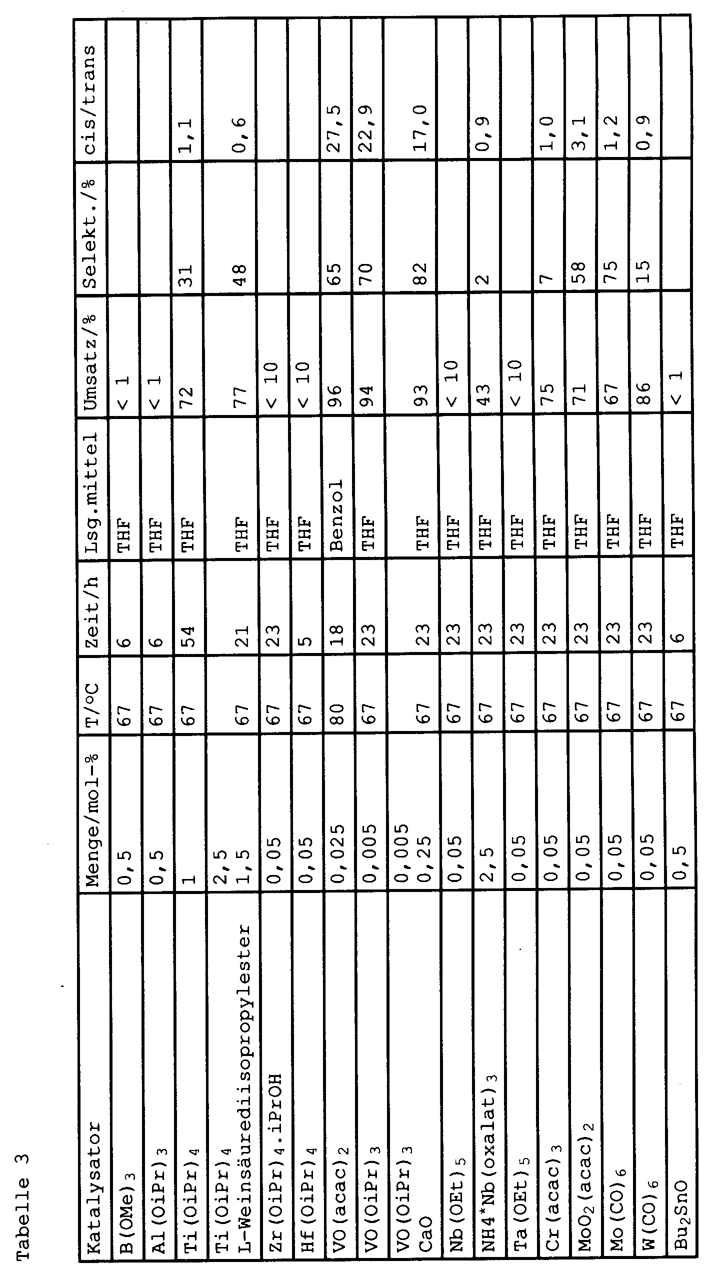

### Beispiel 5

### Umlagerung von cis-2,3-Epoxycyclopentanol zu Glutaroin mit Dikobaltoctacarbonyl und Säure

3 g rohes 2,3-Epoxycyclopentanol (Gehalt an Epoxycyclopentanol ca. 90 % davon ca. 95 % cis) wurden in 20ml trockenem Toluol gelöst. 0,3 g festes Dikobaltoctacarbonyl und 0,09 g p-Toluolsulfonsäure wurden dann auf einmal zugegeben und das Gemisch 21 h bei 50°C unter Stickstoff gerührt. Nach dieser Zeit waren ca. zwei Drittel des Epoxyalkohols umgesetzt und die Selektivität zu Glutaroin betrug 70 %. Es wurden noch 0,3 g festes Dikobaltoctacarbonyl zugegeben und weitere 5 h bei 50°C gerührt. Das cis-2,3-Epoxycyclopentanol war dann zu 93 % umgesetzt. Das trans-2,3-Epoxycyclopentanol bleibt praktisch unverändert. Die Selektivität zu Glutaroin betrug 52 %. Die Reaktionsmischung wurde dann mit Wasser extrahiert. Man erhält eine fast kobaltfreie Lösung von Glutaroin in Wasser.

### Beispiel 6

### Umlagerung von cis-2,3-Epoxycyclopentanol zu Glutaroin mit Dikobaltoctacarbonyl

3 g rohes 2,3-Epoxycyclopentanol (Gehalt an Epoxycyclopentanol ca. 88 %, davon ca. 95 % cis) wurden in 20ml trockenem Benzol gelöst. 0,18 g festes Dikobaltoctacarbonyl wurden dann auf einmal zugegeben und das Gemisch 24 h bei 50°C unter Stickstoff gerührt. Nach dieser Zeit waren ca. 40 % des cis-2,3-Epoxycyclopentanols umgesetzt und die Selektivität zu Glutaroin betrug 80 %.

### Beispiel 7

### Umlagerung von cis-2,3-Epoxycyclopentanol zu Glutaroin mit Natriumtetracarbonylkobaltat und Säure

Natriumtetracarbonylkobaltat (als THF-Lösung) wurde nach der Methode von Edgell und Lyford (Inorg. Chem., 9, 1932 bis 1933 (1970)) aus 1,68 g Dikobaltoctacarbonyl, 2,5 g NaOH und 20 ml THF hergestellt. 5 g rohes 2,3-Epoxycyclopentanol (Gehalt an Epoxycyclopentanol ca. 70 %, davon ca. 95 % cis) und 0,55 g p-Toluolsulfonsäure wurden in 20 ml Toluol gelöst, entgast und auf 60°C erwärmt. Es wurden dann 15 ml der Natriumtetracarbonylkobaltatlösung zugegeben und 43 h bei 60°C gerührt. Umsatz: 42 %, Selektivität: 85 % (laut GC).

### Beispiel 8

### Umlagerung von cis-2,3-Epoxycyclohexanol zu Adipoin (2-Hydroxycyclohexanon)

3 g rohes 2,3-Epoxycyclohexanol (Gehalt an Epoxycyclohexanol ca. 50 %, davon ca. 95 % cis), 0,3 g Dikobaltoktacarbonyl und 0,09 g p-Toluolsulfonsäure wurden in 20 ml Toluol gelöst und 21 h bei 50°C unter Stickstoff gerührt. Umsatz an cis-2,3-Epoxycyclohexanol: 95 %; Selektivität zu Adipoin: 80 %.

### Vergleichsbeispiel 9 (nach J. Org. Chem., 36, 3135-8 (1971) Umlagerung von 2,3-Epoxycyclopentanol zu Glutaroin mit LiBr/RbBr auf Al₂O₃

20 g Epoxycyclopentanol (Gehalt an Epoxycyclopentanol ca. 70 %, davon ca. 95 % cis) wurden langsam verdampft und bei 270°C (5 mbar) über LiBr/RbBr auf Al₂O₃ geleitet 14,1 g LiBr und 15,9 g RbBr auf 90 g Al₂O₃; literaturbekannter Katalysator für die Gasphasenisomerisierung von Epoxiden zu Ketonen). Das Destillat (19,2 g) enthielt ca. 10 % Glutaroin. Das Hauptprodukt war Cyclopenten-2-on.

### Vergleichsbeispiel 10 (nach J. Am. Chem. Soc., 77, 5083-9 (1955) Umlagerung von 2,3-Epoxycyclopentanol zu Glutaroin mit Magnesiumbromidetherat

Magnesiumbromidetherat wird in der Literatur als Reagenz der Wahl zur Isomerisierung von Epoxiden zu Ketonen beschrieben (J. Am. Chem. Soc., 77, 5083 bis 5089 (1955)). 2 g Epoxycyclopentanol (Gehalt an Epoxycyclopentanol ca. 90 %, davon ca. 95 % cis) wurden in 10 ml Diethylether gelöst und 3,4 g Magnesiumbromidetherat dazugegeben. Nach 30 Minuten bei 20°C war das Eddukt vollständig umgesetzt. Es bildeten sich hauptsächlich nicht flüchtige Produkte. Glutaroin entstand nur in Spuren.

### Vergleichsbeispiel 11 (nach J. Am. Chem. Soc., 77, 6525-32 (1955) Umsetzung von 2,3-Epoxycyclopentanol zu Glutaroin mit Bortrifluoridetherat

Bortrifluoridetherat wird ebenfalls in der Literatur als Reagenz zur Isomerisierung von Epoxiden zu Ketonen mehrfach beschrieben
Wenn Lösungen von 2,3-Epoxycyclopentanol in Toluol mit 0,1 bis 10 mol-% Bortrifluoridetherat bei Raumtemperatur behandelt werden, scheiden sich nach kurzer Zeit zähflüssige Polymere ab (Vorsicht! Stark exotherme Reaktion). Glutaroin konnte als Produkt nicht nachgewiesen werden.

### Vergleichsbeispiel 12 (nach Houben-Weyl, Bd. VII/2a, 932-42 (1973), Bd VI/3, 431-41 (1965) und Bd. VII/1, 237-9 (1954) Versuche zur Umlagerung von 2,3-Epoxycyclopentanol mit anderen literaturbekannten Katalysatoren

Folgende Katalysatoren, die zum Teil für die Isomerisierung von Epoxiden zu Ketonen in der Literatur beschrieben sind, erwiesen sich für die Isomerisierung von 2,3-Epoxycyclopentanol zu Glutaroin als inaktiv: Co₂(CO)₆(PBu₃)₂, RhCl(PPh₃)₃, Mo(CO)₆, RuCl₂(PPh₃)₃, RhH(CO)(PPh₃)₃, LiBr/HMPA, LiClO₄, RuH₂(PPh₃)₄, Mn₂(CO)₁₀, Pd auf Träger, Pd(PPh₃)₄, Rh₆(CO)₁₆ und ZnCl₂.

## Patentansprüche

1. Verfahren zur Herstellung von α-Hydroxyketonen der allgemeinen Formel I 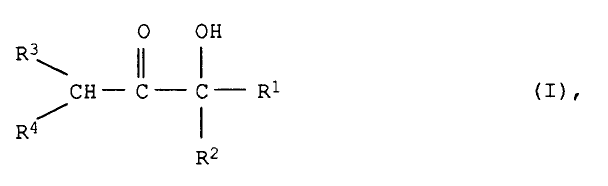 in der
R¹,R²,R³,R⁴ Wasserstoff und C₁- bis C₈-Alkyl oder
R¹ und R³ gemeinsam eine gegebenenfalls ein- bis dreifach durch Methyl und/oder Ethyl substituierte C₂- bis C₁₀-Alkylenkette und
R⁵ Wasserstoff, Methyl und Ethyl
bedeuten, aus 2,3-Epoxialkoholen der allgemeinen Formel II 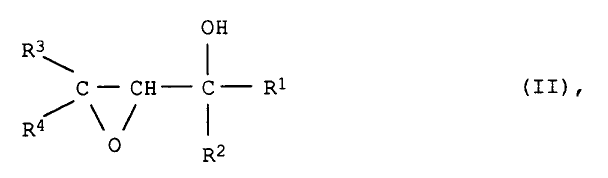 in der R¹, R², R³, R⁴, R⁵ und n die obengenannten Bedeutungen haben, bei Temperaturen von (-10) bis 120°C und Drücken von 0,01 bis 20 bar in Gegenwart eines Katalysators, dadurch gekennzeichnet, daß man als Katalysator eine Hydridokobaltverbindung einsetzt.

2. Verfahren zur Herstellung von α-Hydroxyketonen nach Anspruch 1, dadurch gekennzeichnet, daß man als Hydridokobaltverbindungen solche der allgemeinen Formel HCoL₄, in der L für CO, Tri-C₁- bis C₈-alkylphosphite, Arylphosphite, PF₃ und Tris-(pentafluorphenyl)-phosphin steht, einsetzt.

3. Verfahren zur Herstellung von α-Hydroxyketonen nach Anspruch 1, dadurch gekennzeichnet, daß man die 2,3-Epoxialkohole II durch Umsetzung von 3-Hydroperoxialkenen der allgemeinen Formel III 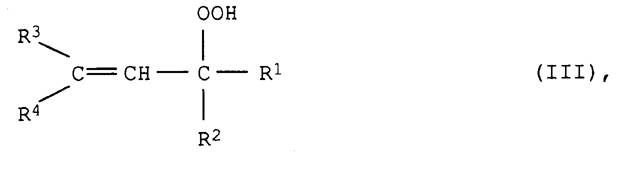 in der
R¹,R²,R³,R⁴ Wasserstoff und C₁- bis C₈-Alkyl oder
R¹ und R³ gemeinsam eine gegebenenfalls ein- bis dreifach durch Methyl und/oder Ethyl substituierte C₂- bis C₆-Alkylenkette und
R⁵ Wasserstoff, Methyl und Ethyl
bedeuten, bei Temperaturen von (-20) bis 120°C und Drücken von 0,01 bis 20 bar in Gegenwart eines Katalysators einer Verbindung eines Übergangsmetalls der Gruppen IVB, VB und VIB durchführt.

4. Verfahren zur Herstellung von α-Hydroxyketonen nach Anspruch 1, dadurch gekennzeichnet, daß man Alkene der allgemeinen Formel IV 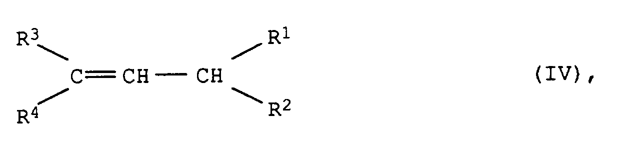 in der
R¹,R²,R³,R⁴ Wasserstoff und C₁- bis C₈-Alkyl oder
R¹ und R³ gemeinsam eine gegebenenfalls ein- bis dreifach durch Methyl und/oder Ethyl substituierte C₂- bis C₆-Alkylenkette und
R⁵ Wasserstoff, Methyl und Ethyl
bedeuten, mit Sauerstoff oder Sauerstoff enthaltenden Gasgemischen bei Temperaturen von 20 bis 120°C zu den 3-Hydroperoxialkenen III und diese bei Temperaturen von (-20) bis 120°C und Drücken von 0,01 bis 20 bar in Gegenwart eines Katalysators einer Verbindung eines Übergangsmetalls der Gruppen IVB, VB und VIB zu den 2,3-Epoxialkoholen II umsetzt.
